# EUROPÄISCHE PATENTANMELDUNG

(11) **EP 3 388 415 A1**
(43) Veröffentlichungstag der Anmeldung: **17.10.2018**
(21) Anmeldenummer: 17165998.0
(22) Anmeldetag: 11.04.2017
(51) Int. Cl.: C07C 45/50, C07C 47/33, C07C 47/43

(54) **VERFAHREN ZUR HYDROFORMYLIERUNG VON CYCLOOCTADIEN UNTER EINSATZ EINES TETRAPHOSPHITS**

(71) Anmelder: Evonik Degussa GmbH, 45128 Essen (DE)
(72) Erfinder: DYBALLA, Katrin, Marie, 45657 Recklinghausen (DE); FRANKE, Robert, 45772 Marl (DE); FRIDAG, Dirk, 45721 Haltern am See (DE); HESS,Dieter, 45770 Marl (DE); SELENT, Detlef, 18059 Rostock (DE); BÖRNER, Armin, 18059 Rostock (DE)

(57) **Zusammenfassung**

Verfahren zur Hydroformylierung von Cyclooctadien (COD) unter Einsatz eines Tetraphosphit-Liganden der Struktur (1).

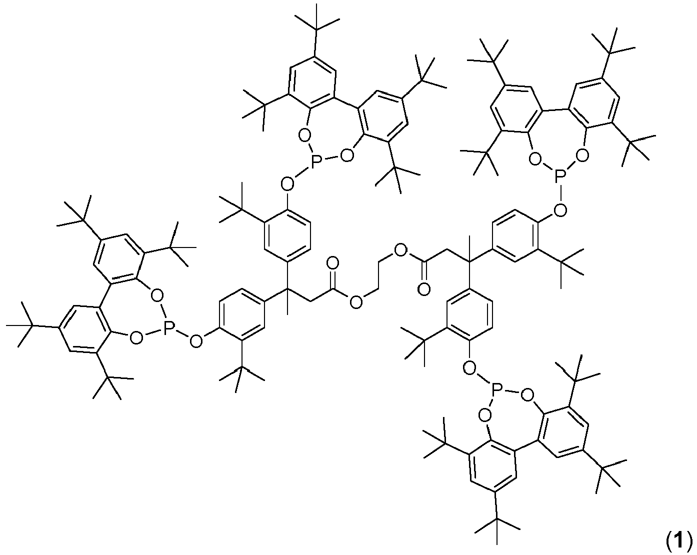

## Beschreibung

Die Erfindung betrifft ein Verfahren zur Hydroformylierung von Cyclooctadien (COD) unter Einsatz eines Tetraphosphits.

Phosphorhaltige Verbindungen spielen als Liganden in einer Vielzahl von Reaktionen eine entscheidende Rolle. Hierzu zählen Phosphitliganden, also Verbindungen, die P-O-Bindungen enthalten, die in der Hydrierung, Hydrocyanierung und vor allem in der Hydroformylierung Anwendung finden.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in R. Franke, D. Selent, A. Börner, "Applied Hydroformylation", Chem. Rev., 2012, DOI:10.1021/cr3001803.

Der Erfindung lag die Aufgabe zugrunde, ein Verfahren zur Hydroformylierung von Cyclooctadien bereitzustellen, welches einen guten Umsatz an Cyclooctadien liefert.

Die Aufgabe wird gelöst durch ein Verfahren nach Anspruch 1.

Verfahren zur Hydroformylierung von Cyclooctadien umfassend die Verfahrensschritte:
a) Vorlegen von Cyclooctadien;
b) Zugabe eines Komplexes umfassend:
   - ein Metallatom ausgewählt aus: Rh, Ru, Co, Ir, und
   - einen Liganden, welcher die Struktur (1) aufweist: 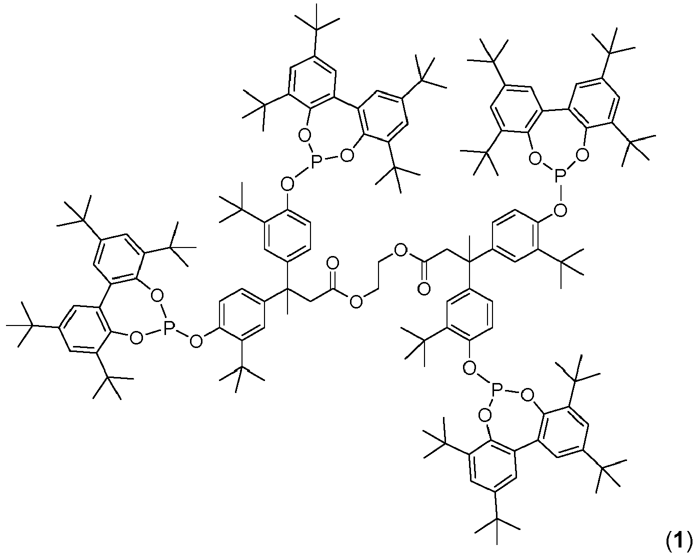 oder
      Zugabe einer Komplexvorstufe umfassend ein Metallatom ausgewählt aus: Rh, Ru, Co, Ir, und einer Verbindung welche die Struktur (**1**) aufweist: 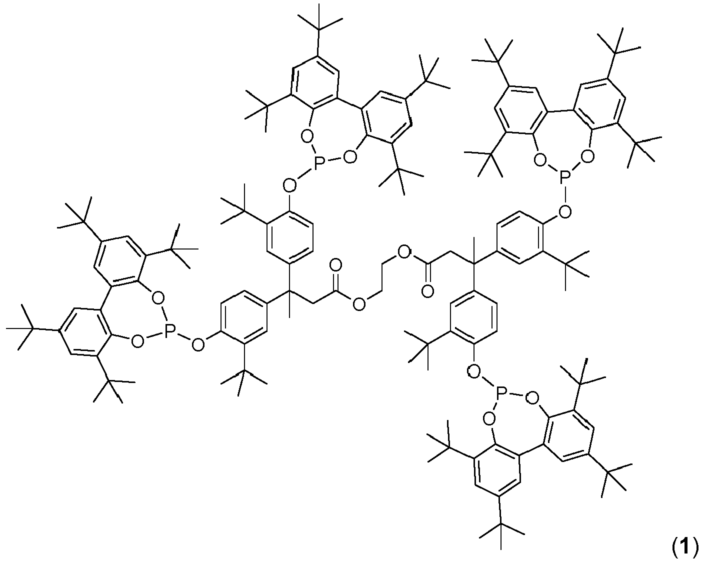
c) Zuführen von H₂ und CO,
d) Erwärmen des Reaktionsgemisches, wobei das Cyclooctadien zu einem Aldehyd umgesetzt wird.

Hierbei können die Verfahrensschritte a) bis d) in beliebiger Reihenfolge erfolgen.

In einer Variante des Verfahrens ist das Metallatom Rh.

In einer Variante des Verfahrens umfasst die Komplexvorstufe Cyclooctadien.

In einer Variante des Verfahrens handelt es sich bei der Komplexvorstufe um [(acac)Rh(COD)]. Hierbei stehen "acac" für Acetylacetonat-Anion und "COD" für Cyclooctadien.

In einer Variante des Verfahrens wird das Reaktionsgemisch im Verfahrensschritt d) auf eine Temperatur im Bereich von 50 °C bis 70 °C erwärmt.

Im Folgenden wird die Erfindung anhand von Ausführungsbeispielen näher erläutert.

### Allgemeine Arbeitsvorschriften

Alle präparativen Arbeiten erfolgten unter Anwendung der Schlenk-Technik mit Argon als Schutzgas. Toluol und Tetrahydrofuran wurden mit einem Pure Solv MD-7 System gereinigt und bis zur Verwendung unter Argon aufbewahrt. Triethylamin wurde vor dem Einsatz unter Argon von Natriumketyl destilliert. Phosphortrichlorid (Aldrich) wurde vor dem Einsatz unter Argon destilliert. Alle präparativen Arbeiten erfolgten in ausgeheizten Gefäßen.

Die im Folgenden dargestellten Liganden sind kommerziell verfügbar, oder ihre Herstellung in der Literatur beschreiben.

### Ligand 1

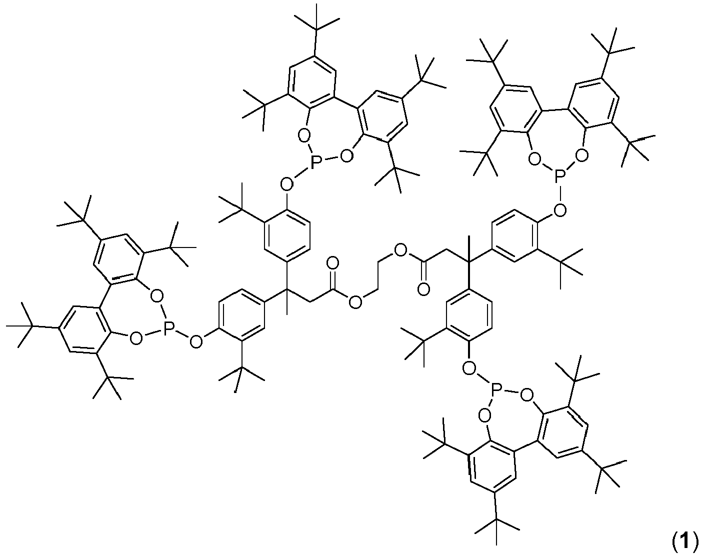

### Ligand 2

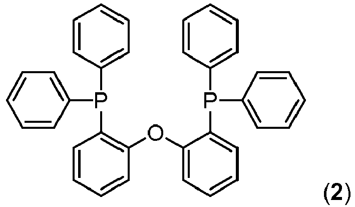

### Ligand 3

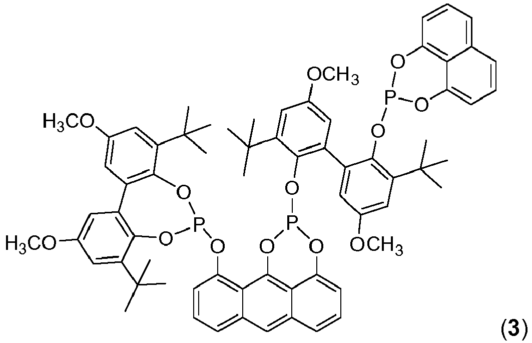

### Hydroformylierung

Die Hydroformylierungsreaktionen wurden in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Das als Lösungsmittel verwendete Toluol wurde mit einem Pure Solv MD-7 System gereinigt und unter Argon aufbewahrt.

Für die Versuche wurden im Autoklaven unter Argonatmosphäre Lösungen der Komplexvorstufe (= Katalysatorvorstufe) und des Liganden gemischt. Als Komplexvorstufe kam [(acac)Rh(COD)] (Umicore, acac = Acetylacetonat-Anion; COD= 1,5-Cyclooctadien) zum Einsatz. Für Versuche mit einer Konzentration von 100 ppm-m Rhodium wurden 5 ml einer 4,32 millimolaren Lösung in den Autoklaven gegeben. Anschließend wurde die einem Verhältnis L/Rh = 2:1 entsprechende Masse des Liganden in 20 ml Toluol gelöst und zugemischt. In eine druckfeste Pipette wurde eingefüllt: 2,69 g (24,86 mmol) COD-1,5. Der Autoklav wurde mit Synthesegas (Linde; H2 (Qualität 5.0) : CO (Qualität 4.7) = 1:1) auf einen Druck von 42 bar gebracht und auf 60 °C aufgeheizt. Nach Erreichen der Reaktionstemperatur wurde das Diolefin in den Autoklaven gepresst. Die Reaktion wurde bei konstantem Druck von 50 bar (Nachdruckregler der Fa. Bronkhorst, NL) ausgeführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm.

Die Ergebnisse der Hydroformylierungsversuche sind in der nachfolgenden Tabelle zusammengestellt. Der angegeben Umsatz umfasst hierbei sowohl Monoaldehyde wie auch Dialdehyde.

Standard-Versuchsbedingungen: [Rh]= 0,717 x 10⁻⁴ M, Rh/Ligand/COD-1,5 = 1:2:1151, Lösungsmittel Toluol.

**Tabelle 1:**

| Ligand | p [bar] | T [°C] | t [h] | Umsatz COD-1,5 [%] |
|---|---|---|---|---|
| **1*** | 50 | 60 | 4 | 99 |
| **2** | 50 | 60 | 4 | 1 |
| **3** | 50 | 60 | 4 | 31 |

| | | | | |
|---|---|---|---|---|
| *) erfindungsgemäßes Verfahren | | | | |

Wie die Versuchsergebnisse zeigen, wird die Aufgabe durch das erfindungsgemäße Verfahren gelöst.

## Patentansprüche

1. Verfahren zur Hydroformylierung von Cyclooctadien umfassend die Verfahrensschritte:
a) Vorlegen von Cyclooctadien;
b) Zugabe eines Komplexes umfassend:
- ein Metallatom ausgewählt aus: Rh, Ru, Co, Ir, und
- einen Liganden, welcher die Struktur (1) aufweist: 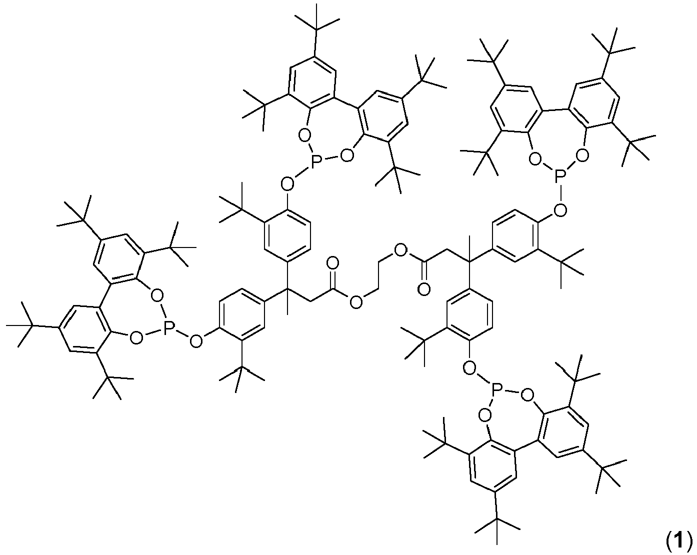 oder
Zugabe einer Komplexvorstufe umfassend ein Metallatom ausgewählt aus: Rh, Ru, Co, Ir, und einer Verbindung welche die Struktur (**1**) aufweist: 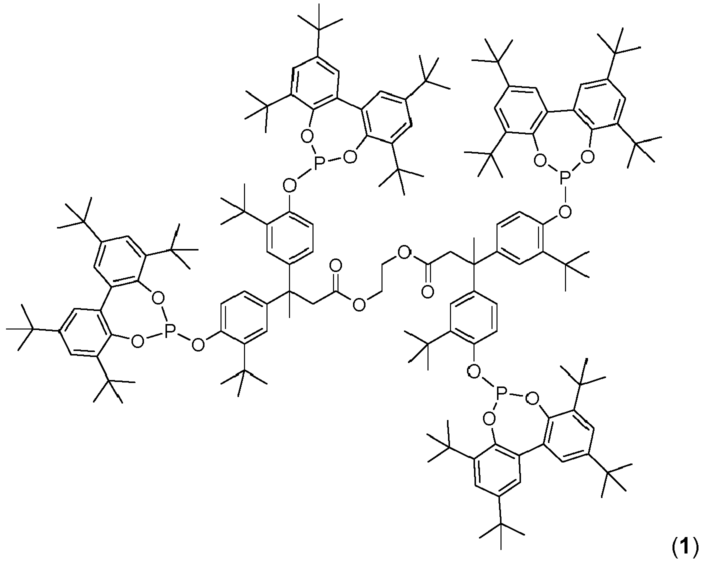
c) Zuführen von H₂ und CO,
d) Erwärmen des Reaktionsgemisches, wobei das Cyclooctadien zu einem Aldehyd umgesetzt wird.

2. Verfahren nach Anspruch 1,
wobei das Metallatom Rh ist.

3. Verfahren nach einem der Ansprüche 1 oder 2,
wobei die Komplexvorstufe Cyclooctadien umfasst.

4. Verfahren nach einem der Ansprüche 1 bis 3,
wobei es sich bei der Komplexvorstufe um [(acac)Rh(COD)] handelt.

5. Verfahren nach einem der Ansprüche 1 bis 4,
wobei das Reaktionsgemisch im Verfahrensschritt d) auf eine Temperatur im Bereich von 50 ° C bis 70 °C erwärmt wird.
